(19)

Europäisches Patentamt
European Patent Office
Office européen des brevets

(11) **EP 4 749 634 A1**

(12) **EUROPEAN PATENT APPLICATION**

(43) Date of publication:
27.05.2026 Bulletin 2026/22

(21) Application number: 24214831.0

(22) Date of filing: 22.11.2024

(51) International Patent Classification (IPC):
G16H 20/00 (2018.01)        G16H 30/40 (2018.01)
G16H 50/20 (2018.01)        G16H 50/70 (2018.01)
A61B 5/00 (2006.01)         G06T 7/00 (2017.01)

(52) Cooperative Patent Classification (CPC):
G16H 30/40; A61B 5/4566; G16H 20/00;
G16H 50/20; G16H 50/50; G16H 50/70

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Inoteqia Health Tech
2045 Ain Zaghouan Tunis (TN)

(72) Inventors:
• Moez Touil, Mohamed
1082 Tunis (TN)

• Ksantini, Riadh
Zallap (BH)
• Mbarek, Eya
Nabeul (TN)
• Makhlouf, Aziza
Tunis (TN)
• Trabelsi, Fatma Zahra
Tunis (TN)
• Tbarki, Khaoula
Tunis (TN)

(74) Representative: Dupuis-Latour, Dominique
Bardehle Pagenberg SPE SAS
SO Square Opéra
5, rue Boudreau
75009 Paris (FR)

(54) **METHOD FOR PROCESSING PHYSIOLOGICAL DATA OF A SPINE OF A PATIENT**

(57) This invention presents a new system designed for the comprehensive detection, classification, identification, next-state prediction, and real-time monitoring of spinal pathologies using machine learning based on clinical data and medical imaging. It involves detecting pathologies, recognizing their specifics, recommending treatments, and predicting future states. The system integrates advanced imaging to create a 3D spine model, assesses muscle quality, and confirms initial evaluations with clinical data. Predictive analytics estimate disease progression and treatment outcomes. Additionally, a digital twin of the spine, with integrated sensors for real-time monitoring, enables continuous data collection and timely interventions. This comprehensive approach enhances diagnostic accuracy, treatment planning, and patient management, offering a robust solution for spinal pathology evaluation and prediction.

[Figure 1]

EP 4 749 634 A1

**Description**

BACKGROUND OF THE INVENTION

*Field of the invention*

The present invention generally relates to advanced medical devices and systems, specifically focusing on providing comprehensive assessment, prediction, and analysis information for spine conditions.

The spine is a complex structure composed of bones, ligaments, tendons, and muscles. It supports the body, protects the spinal cord, and allows for movement. Spinal pathologies refer to the set of diseases affecting the spine. Spine is composed of a stack of vertebrae; this part of the body is particularly fragile as it supports the head and transmits the entire body weight to the hips. Spinal pathologies are divided into three main groups: degenerative pathologies, tumor pathologies, and traumatic pathologies.

The first group includes lumbar and cervical disc hernias, sciatica, cervical myelopathy, and scoliosis. The second group mainly includes metastases from solid cancers or haematological diseases. As for the traumatic pathologies of the spine, they primarily concern lumbar and thoracic fractures, and vertebral compression.

In the medical field, many significant challenges are posed. Among these challenges we can mention:

- *misidentification*: frequent misidentification can have a substantial influence on patient health and treatment outcomes, making it more difficult to accurately detect and identify spine diseases;
- *over-reliance on medical images*: when making a spine pathology analysis, doctors frequently rely too much on medical images and occasionally ignore important clinical and personal details about their patients;
- *incomplete patient records*: when vital information is lost, patient records may become incomplete, which compromises the precision of future monitoring and treatment planning;
- *unnoticed disease progression*: spine pathologies evolve so quickly, it is possible for them to go unidentified until they have progressed to a point where they pose a threat to the patient's health.

Many studies have utilized artificial intelligence to detect pathologies through MRI or Computerized Tomography (CT) images. These images are pre-processed and analyzed by models to classify and grade spine pathologies. However, theses methods depend only on imaging data, neglecting the patient's personal information, which is crucial.

The present invention aims to fill this gap by incorporating personal data into the analysis. We aim to identify spine pathologies, recommend tailored treatments, and predict the future progression of the detected conditions.

SUMMARY OF THE INVENTION

Essentially, the invention provides a new system to detect, classify, identify the spine pathology, and predict the future state of the detected spinal condition based on machine learning algorithms using two types of data: clinical data and medical imaging.

Spinal pathologies detection and prediction system involves several processes, including: pathology detection, pathology specification recognition, treatment recommendation, next-state prediction of the spinal pathologies, image segmentation, 3D reconstruction of the spine, results comparison and treatment update, and doctor check and validation.

The spine pathologies detection process aims to detect the pathology in the spine of the patient using clinical exam information collected from previous patients. The detected pathology will be detailed using the pathology specification recognition process, that means this latter can for example locate the pathology, detect the side, and predict the type.

After that, needed treatment based on predicted information will be recommended as a result of treatment recommendation process and should be checked by the doctor. This process helps doctors and simplifies the initial evaluation for pathologies during the first consultation without immediately requiring imaging.

Also, the system includes an advanced imaging component. After requiring the patient's images, it generates a 3D representation of the spine, including muscles and nerves.

Furthermore, the system can identify pathologies and assess muscle quality. This thorough analysis is employed to confirm or adjust the initial evaluation using the clinical data, providing critical insights into any abnormalities and the overall health of the musculoskeletal system. This advanced analysis of the medical image improves the accuracy of the assessment and treatment planning.

Moreover, the system includes predictive analytics capabilities. By analyzing the current patient data (both clinical data and medical images), it can predict the progression of the condition. It can estimate whether the state will improve or worsen over time, forecast the likelihood of recurrence over a specific period, and evaluate potential outcomes regardless of the treatment received.

Finally, a digital twin of the patient's spine will be created.

This includes integrating sensors into the patient's spine to enable real-time monitoring. The sensors provide continuous data collection, notifying healthcare professionals of any changes or potential issues. This continuous monitoring enables prompt interventions and the refinement of predictive models, thereby enhancing the system's capability to accurately predict the patient's future health trajectory.

More specifically, the present invention provides for a method for processing physiological data of a spine of a patient, comprising the steps of: a) collecting and processing clinical data related to the patient's spine; b) collecting and processing medical imaging data related to the patient's spine; and c) reconciling processed clinical data and medical imaging data, for issuing data representative of a status of the patient,

According to the invention, said processing of clinical data in step a) includes:

a1) pre-processing said clinical data and applying, to pre-processed clinical data, a first spinal pathology detection algorithm;

a2) pre-processing said clinical data and applying, to pre-processed clinical data, a first pathology characterization algorithm, specific to the spinal pathology detected in step a1); and

a3) applying, to data issued by said first algorithms in steps a1) and a2), a therapeutic recommendation algorithm.

Further, in step b) processing of medical imaging data includes:

b1) providing, from said medical imaging data, a structural 3D digital model representative of a morphology of the patient's spine;

b2) providing, from said medical imaging data, a predictive digital model representative of a future condition of the patient's spine;

b3) pre-processing said medical imaging data and applying, to pre-processed medical imaging data, a second spinal pathology detection algorithm; and

b4) pre-processing said medical imaging data and applying, to pre-processed medical imaging data, a second pathology characterization algorithm, specific to the spinal pathology detected in step b3).

Still further, in step c) said reconciling includes:

c1) applying a matching algorithm receiving as inputs the results jointly issued as outputs by said first and second detection algorithms, said first and second pathology characterization algorithms, and said therapeutic recommendation algorithm, respectively applied in steps a1)-a3) and b3)-b4); and

c2) automatically validating or correcting a recommended treatment issued by said therapeutic recommendation algorithm and outputting a validation/invalidation signal to the physician, depending of the result issued by said matching algorithm applied in step c1).

The specific characterization of a spinal pathology may in particular includes determining: a subtype of a pathology, a localization of a pathology with respect to the patient's spine; and a lateralization of a pathology.

BRIEF DESCRIPTION OF THE DRAWINGS

The foregoing and other objects, aspects and advantages of the invention will be better understood from the following detailed description of a preferred embodiment of the invention with reference to the appended drawings, in which the same numerals refer to identical or functionally similar features over the different figures. These illustrations are intended for clarification purposes only and are not meant to limit the scope of the present disclosure.

**Figure 1** illustrates the architecture of spinal pathologies detection and prediction system.
**Figure 2** presents an example of pathology statistic dashboard.
**Figure 3** describes the spinal pathologies detection process.
**Figure 4A** shows an example of original data.
**Figure 4B** shows the encoded data of the example shown in **Figure 4A.**
**Figure 5** describes the steps of pathology specification recognition process of the detected disease.
**Figure 6** describes the architecture of our proposed method one-against-all multi-class SVM one-class classification.
**Figure 7** shows the architecture of our proposed method one-against-one multi-class SVM one-class classification.
**Figure 8** presents a treatment recommendation process steps.
**Figure 9** shows the selected features for treatment recommendation using Explainable Boosting Classifier.
**Figure 10** presents medical images processing process architecture.

**Figure 11** illustrates the steps of medical images preprocessing proposed approach.

**Figure 12** illustrates the sequence-to-sequence LSTM model for predicting a sequence of T future landmarks from the given k reference points.

**Figure 13** shows the LSTM cell used in the next-state prediction process.

**Figure 14** describes the results verification and validation process.

DETAILED DESCRIPTION OF A PREFERRED EMBODIMENT OF THE INVENTION

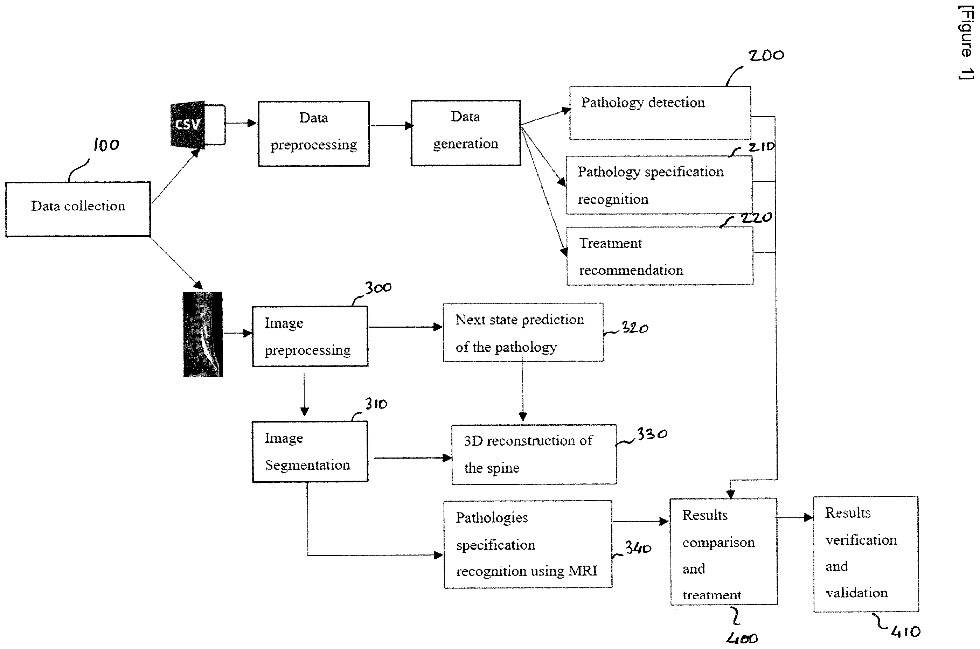

Referring now to the drawings, **Figure 1** illustrates the architecture of spinal pathologies detection, classification, and prediction system. It includes, as mentioned above, seven primary processes, which may be brought together in three groups:

- the first group, based on clinical data, involves three processes: pathology detection 200, pathology specification recognition 210, and treatment recommendation 220;
- the second group, requiring medical images, encompasses the following processes: next-state prediction of the pathology 320, 3D reconstruction of the spine 330, and pathology specification recognition using medical imaging 340; and
- the third group is an extensive comparison 400 between the results obtained in the first and the second parts, enabling the doctor to verify and validate the findings (block 410).

**Data collection** (block 100)

To collect data from hospitals and clinics, we have prepared a file patient for each spinal pathology. It contains important pathology features, which are recommended by the doctors. For instance, in the case of disc herniation pathology, it is proposed to select patient personal information, disease history, symptomatology and physical exam, etc.

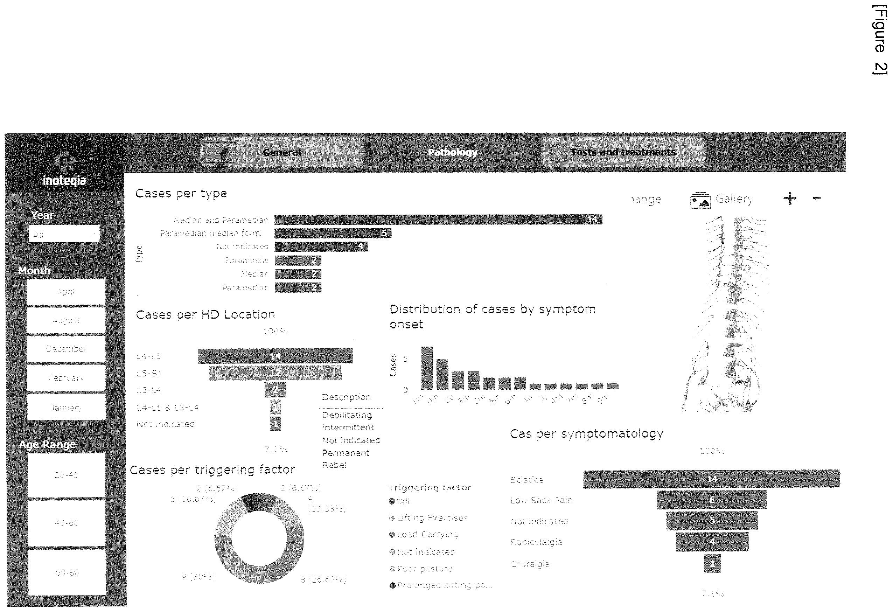

Web and mobile applications have been developed for data collection. The latter has been used by the doctors to automatically save patient pathology information, as mentioned earlier, as well as medical images, and then to follow up the state of the patient. In addition, the application allows the doctor to visualize data statistics, such as general statistics (statistics per type, location, factors, etc), pathology, test and treatments statistics, e.g. presented as a dashboard, as shown in **Figure 2.** Moreover, the results of the seven above-mentioned processes 200-220, 320-340 and 400 have been deployed in the application. So, these processes help the doctors in making their decisions.

**Spinal pathologies detection process** (block 200)

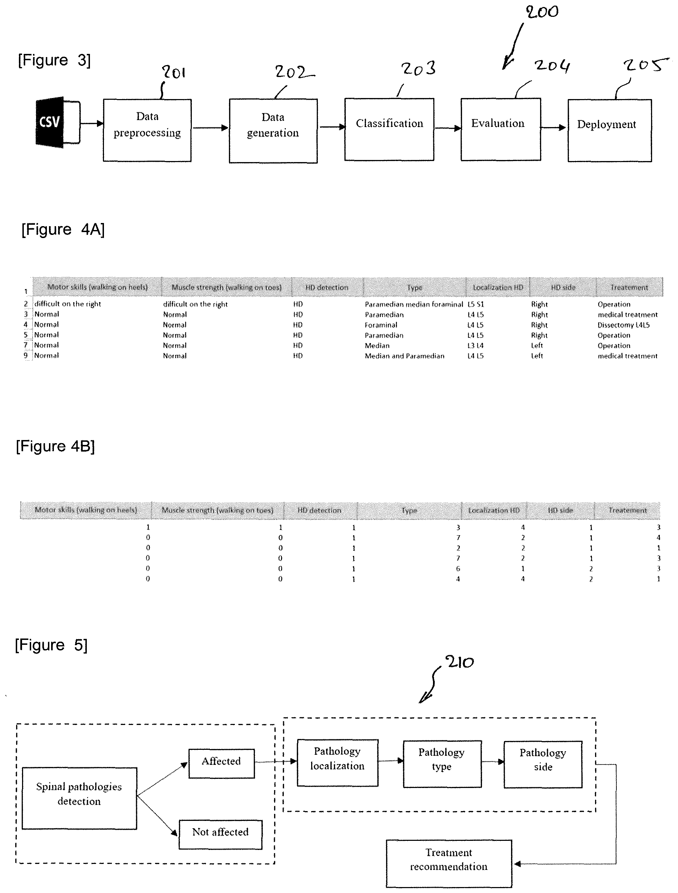

The spinal pathologies detection process aims to detect the pathology using a vector of collected features. This process is composed of five main steps 201-205, as shown in **Figure 3.**

For the disc herniation pathology, we started by discriminating between an affected patient and a normal patient using clinical data. This latter comprises 35 patients, with 30 samples showing affected conditions and 5 samples considered normal. Each patient record includes 30 attributes (features), such as age, sex, occupation, onset date of illness, and clinical exam details.

Some features of the used dataset are categorical **(Figure 4A),** making them unreadable for classification algorithms. For this reason, a Label Encoder was utilized as a preprocessing tool to convert categorical data into numerical data, as presented in **Figure 4B.**

Given that the used dataset is very small, we used a Conditional Tabular Generative Adversarial Network (CTGAN) to generate artificial data based on 30 real samples. Data generation is used as a temporary step until all necessary data is collected. Using this latter, we generated 1970 samples and we obtained a new dataset which is composed of 2000 affected samples and 5 normal samples. So, we can clearly notice that the used dataset is imbalanced, where the number of affected samples is much greater than the normal one. For this reason, in the classification step, one-class classifiers have been chosen, as they handle the problem of unbalanced data. Among these classifiers we can mention: One-class Support Vectors Machine (OSVM), Local Outlier Factor (LOF), One-class Deep Support Vector Data Description (OCSVDD), Mahalanobis One-Class Support Vector Machine (MOSVM), Covariance Guided Support Vector Machine (COSVM), and the incremental versions iOSVM, iMOSVM and iCOSVM, respectively, of models OSVM, MOSVM and COSVM.

Generally speaking, machine learning classification methods are composed of two steps: training and testing steps. One-class classification algorithm uses only target data in the training step but in the testing step, both target and outliers are used. A holdout method has been used to split data into two sets, the training set is used to build the model, which is composed of 80% of only target data (1600 samples), and the testing set is used to test the model and to evaluate its performance, which is composed of the rest of the target class (20%: 400 samples) and all outlier samples (5 samples).

An extensive comparative evaluation between incremental and batch one-class classifiers has been performed, to demonstrate the superiority of incremental learning in spine pathology detection.

A majority voting method is used between three machine learning, which are OSVM, LOF and OCSVDD, in order to enhance the performance of the three mentioned models. They have been trained on the same used dataset. In the final result, each model casts its vote for the outcome, and the final results are determined by the majority's decision.

For one-class classifiers, generally, Area Under the ROC Curve (AUC) is used as a measurement of performance. In fact, it is expressed as a function of the true-positive rate versus the variation of the false-positive rate, where the true-positive means a positive instance (affected patient) classified correctly as positive, and if a negative instance classified incorrectly as positive, it is considered as false-positive.

To evaluate the performance of our proposed spine pathologies detection method, we use AUC as an experimental protocol. In this invention we choose to demonstrate the results of one spine pathology, which is disc herniation pathology.

**Table 1** below illustrates the obtained results of disc herniation detection process in terms of AUC using one-class classifiers.

We can clearly see from this table, that one-class iCOSVM provides better results in term of AUC, that is expected because iCOSVM has the advantage of incrementally emphasizing low-variance directions while classifying data.

Moreover, it is obvious that the incremental learning provides better results compared to batch learning. More specifically, from Table 1 we can clearly remark, that iOSVM, iMOSVM and iCOSVM give larger AUCs better than OSVM, MOSVM and COSVM, respectively. That is explained by that the incremental approach is adaptable to dynamic or extensive datasets, allowing it to incorporate new data without the need to retrain on the entire previous dataset. Additionally, it operates without requiring access to the original data.

Also, from Table 1 we can clearly see that the results of majority voting method are better than OSVM and LOF results and closer to OCSVDD.

[Table 1] One-class classifiers result **(best results in boldface)**

| Method | AUC (%) |
|---|---|
| OSVM | 89.12 |
| Local Outlier Factor (LOF) | 99.37 |
| One-class Deep SVDD (OCSVDD) | 74.37 |
| Majority voting (OSVM/LOF/OCSVDD) | 98 |
| MOSVM | 88.6 |
| COSVM | 99.7 |
| iOSVM | 98.62 |
| iMOSVM | 98.65 |
| **iCOSVM** | **100** |

**Spinal Pathology specification recognition process** (block 210)

As presented in **Figure 5,** the first process of our method is to detect the spinal pathology, so it is necessary to delve deeper into the patient's condition by extracting additional details about the identified pathology, subsequently, such as, to involve accurately locating the pathology in the spine, discerning the affected side (if applicable) and predicting the specific type of the detected pathology.

Pathology specification recognition process is composed of the same steps as pathology detection process: data preprocessing, data generation, classification, evaluation and deployment as shown in **Figure 3.**

It allows doctor to locate the pathology, and to define its type and its side (if applicable) based on machine learning algorithms.

In fact, since we expect real dataset to be imbalanced (e.g., for the type of disc herniation where one type might be more prevalent than others), we propose a novel method, one-class multi-class classification based on One-class SVM (OSVM) to handle the problem of unbalanced data. Our proposed method is based on the same principle of the original multi-class classification, which is composed of binary SVM using the techniques of One-versus-One and One-versus-All. Our proposed model is composed of two techniques: One-Against-All Multi-class One-class SVM (OAA-MCOSVM), and One-Against-One Multi-class One-class SVM (OAO-MCOSVM).

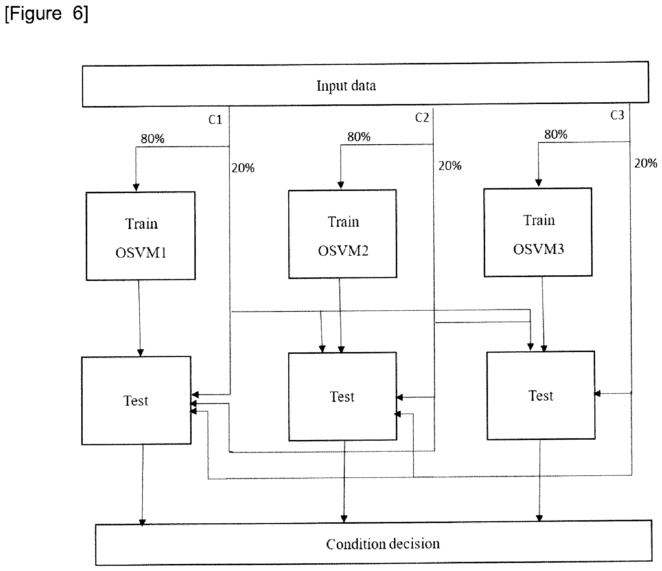

In **Figure 6,** the flow chart for OAA-MCOSVM model is presented.

The OAA-MCOSVM method needs $K$ iterations, where $K$ is the number of classes. At each iteration, a one class is separated from others, to be considered as the target class, which is labeled by +1, and the rest of classes are considered as outliers and labeled by -1. Then, an OSVM classifier will be built. This latter requires only the target class in the training step, but in the test step it takes into consideration both classes target and outlier categories. In OAA-MCOSVM, $K$ one-class classifiers are evaluated for $K$ classes.

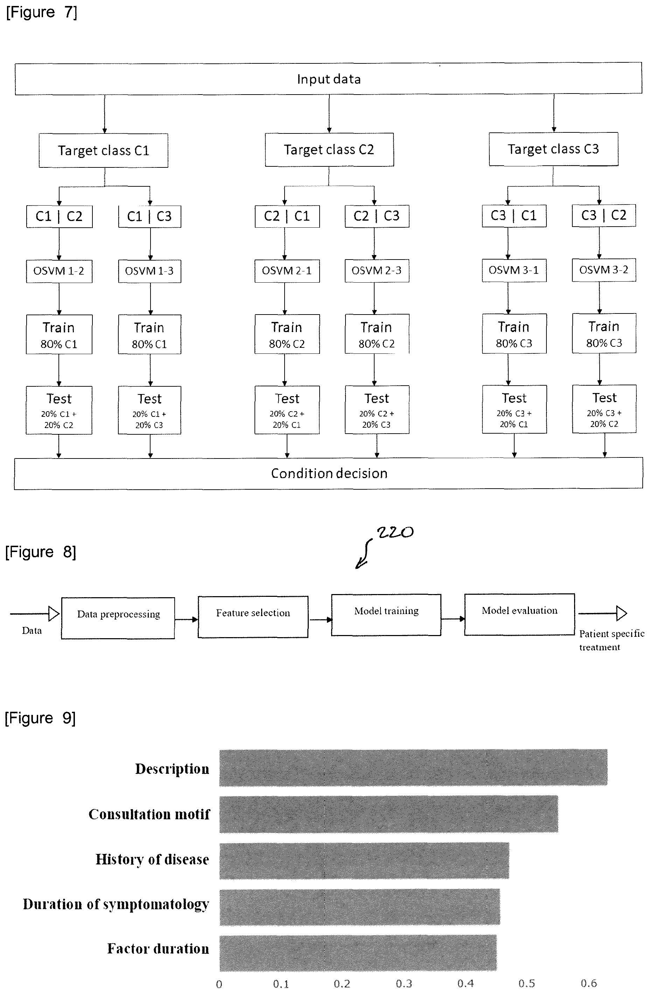

**Figure 7** shows the flow chart of OAO-MCOSVM method.

In The OAO-MCOSVM approach the numbers of needed iterations are always larger than the number of iterations required by the OAA-MCOSVM. In this latter, for $K$ classes, $K$ classifiers are tested, but for OAO-MCOSVM $K$ ($K$-1) predictors are required.

As mentioned $K(K$-1) iterations are necessary. All classes should be represented as target class labeled by +1. Then, $K$-1 one-class classifiers will be built for each target class, where for each classifier, the training set is composed only of target data and the test set is composed by the target and outlier data. Finally, the result is obtained by majority voting of all used classifiers.

As we can clearly see from **Figure 7,** in case of three classes, six iterations are needed. $C_1$, $C_2$ and $C_3$ represent three different target classes. In case $C_1$ is selected as target class, we create two OSVM models, the first one is trained on $C_1$ only and tested on both $C_1$ and $C_2$. The second model concerned $C_1$ versus $C_3$ and uses the same principle of the first model.

This iterative process ensures that each class specific characteristics are captured independently. This approach effectively handles class imbalances, ensuring robust and accurate classification by treating each class in a proper manner.

To evaluate the performance of the proposed classifiers (OAA-MCOSVM and OAO-MCOSVM), we split the data using a Holdout method (which method had been mentioned in the introductory part of the present specification) into two sets, training sets which is composed of 80% of data and the rest of data is used as test set.

Then, Accuracy, Recall, Precision, and F1-Score are computed as to measure the performance of evaluated models. These metrics are defined in equations [Math 1]- [Math4] below, respectively, where True Positive (TP) means that a positive instance is classified correctly as positive; if it is classified incorrectly as negative, it is considered as False Negative (FN). True Negative (TN) represents a negative instance which is classified correctly as negative, if it is classified incorrectly as positive, it is considered as False Positive (FP). $N$ represents the number of samples in our dataset.

[Math 1]

$$Accuracy = \frac{TP + TN}{N}$$

[Math 2]

$$Recall = \frac{TP}{TP + FN}$$

[Math 3]

$$Precision = \frac{TP}{TP + FP}$$

[Math 4]

$$F1 - score = \frac{2 * Precision * Recall}{Precision + Recall}$$

**Treatment recommendation** (block 220)

Treatment recommendation process is to suggest appropriate treatments to the patient based on the detailed analysis of the obtained results of the Spinal Pathology Specification Recognition process.

As seen in **Figure 8,** this latter involves selecting relevant features that influence treatment decisions before training and evaluating the model, ensuring a personalized and accurate treatment plan for each patient. Treatment recommendation process requires the results of the pathology specification recognition process, such as the detected pathology, its type, side and localization.

- **Detected pathology:** the specific type of spinal pathology identified (e.g., disc herniation, spinal stenosis);
- **Type:** the subtype of the pathology (e.g. median herniation, Isthmic spondylolisthesis, central stenosis);
- **Side:** the affected side of the body (e.g. left, right, bilateral);
- **Localization:** the exact location within the spine (e.g. cervical, thoracic, lumbar regions).

For the Treatment recommendation step, not only the results of pathology specification are needed, but also other factors. For example, if the disc herniation pathology is detected, we add some features based on the obtained results of Explainable Boosting Classifier (EBC) **(Figure 9).**

Among these features, we may mention:

1) Symptomatology description: treatments vary for patients with severe, unresponsive pain versus those with tolerable pain;
2) Consultation motif: each complaint may correspond to a different treatment;
3) History of disease: patients with recurring issues might need more aggressive or alternative treatments compared to those with a first-time occurrence;
4) Duration of symptomatology: chronic symptoms may require different approaches compared to acute symptoms.
5) Factor duration: the treatment for a patient who has had a longstanding issue differs from that for a patient who seeks medical help immediately after an incident.

With diverse and extensive data, the correlation between features becomes more relevant, leading to more accurate feature selection and by considering these additional factors, the treatment recommendation process can be tailored to address the unique needs of each patient.

To build a machine learning model for treatment recommendation, we have chosen the same models as above, namely OAA-MCOSVM and OAO-MCOSVM methods. These models are initially tested on all available features, and then on selected features. These tests have shown that using the selected feature only provides better results. To assess the performance of our selected models, we use accuracy, F1-Score, Recall, and precision, as defined above to evaluate the performance of proposed classifiers OAA-MCOSVM and OAO-MCOSVM.

**Medical Image processing** (blocks 300, 310)

As mentioned in the introductory part of the present detailed description,, two types of data are needed in our project, namely, clinical data and imaging data, in order to obtain accurate analysis. Clinical data contains important features about the patient, they give an hypothesis about pathology, its type, the side, suggest treatment, etc., whereas medial images provide critical insights into anatomical and pathological conditions.

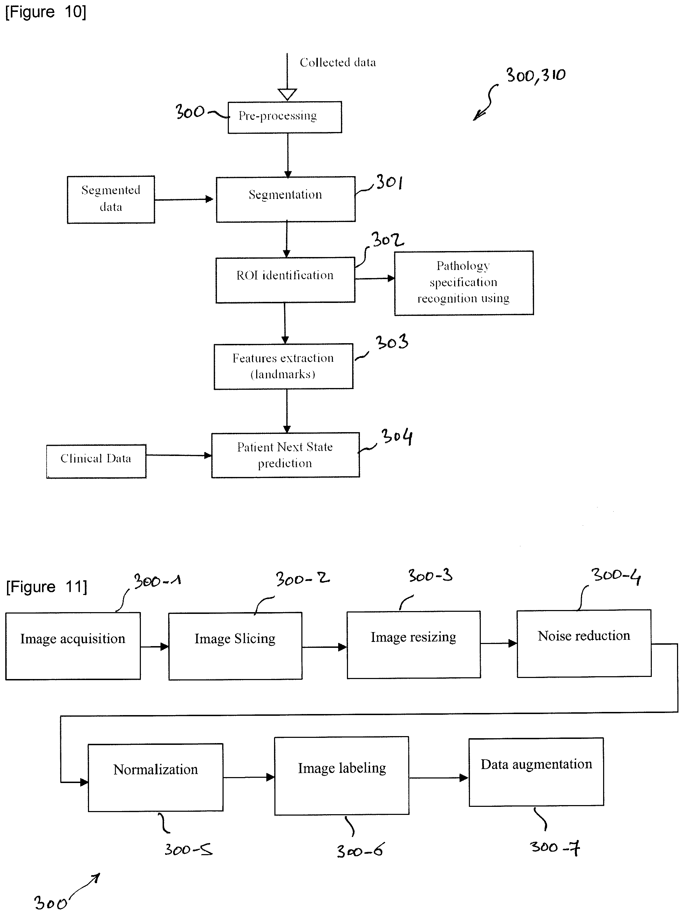

However, to leverage the full potential of medical images, they must first undergo a series of processing steps to be ready for analysis, as summarized in **Figure 10.**

From this figure, we can clearly see that a pre-processing step 300 is applied to the medical imaging collected data to ensure that the images are suitable for subsequent stages, such as segmentation 301, Regions of Interest (ROI) identification 302, feature extraction 303, and prediction 304.

To ensure optimal quality medical images for analysis, we propose in **Figure 11** the main steps needed in the data pre-processing method. These steps include image acquisition 300-1, slicing 300-2, resizing 300-3, normalization 300-4, noise reduction 300-5, annotation 300-6, and augmentation 300-7. Each of these steps is crucial in preparing the data for further analysis and integration with clinical information.

Once the data is properly cleaned and prepared, it is used to determine the masks for sagittal and axial views. Given the limited number of images in the available dataset, we employed an effective data augmentation technique. This latter includes two primary actions: rotation and flipping, which are applied to both the images and their corresponding masks. Following the data augmentation step, annotation and labeling techniques are required to identify and mark regions of interest (ROIs) within the images, such as vertebrae, intervertebral discs (IVDs), and other relevant anatomical structures and anomalies. The obtained result of this step is a JSON file containing the labels of the structures along with the x and y coordinates of the boundaries. From these coordinates, only needed anatomic edge will be extracted. Finally, a feature extraction step will be performed to distinguish between normal and abnormal spine conditions. For example, by measuring features, such as, the width and height of IVDs, we can provide valuable input to machine learning models, enhancing their ability to detect and classify spinal pathologies.

The Image processing process allows to prepare the data for other processes, such as, Patient Next-State Prediction and Pathology Specification Recognition using medical imaging.

### Next-state prediction of the pathology (block 320)

The objective of this process is to develop a novel model for spinal pathologies next-state prediction based on medical imaging. The ability to predict the future state of these pathologies can significantly enhance patient care by enabling early intervention, personalized treatment plans, and better management of chronic conditions.

Our proposed predictive model will leverage advanced Deep Learning (DL) algorithms to provide accurate and reliable predictions, ultimately improving outcomes for patients with spinal disorders.

Two approaches have been used in this process to predict next state of the spine, an Auto-Encoder Convolutional Long Short-Term Memory (AE-ConvLSTM) and a Hierarchical approach which combines Generative Adversarial Network (GAN), Long Short-Term Memory (LSTM), Auto-encoder and Convolutional Neural Network (CNN).

### AE-ConvLSTM method

The AE-ConvLSTM method is a neural network architecture that incorporates CNNs with LSTM networks to handle sequence-to-sequence learning tasks.

This hybrid model has proven to be very competitive in applications involving spatiotemporal data like video processing or time series analysis with spatial components. Notably, it is highly effective in next-frame prediction, making it ideal for predicting future states in dynamic systems. The architecture of this model includes two main components: an encoder and a decoder.

Applying this approach to spinal imaging data analysis could significantly enhance predictive modeling in medical imaging.

To evaluate our predictive spinal imaging sequence model, we utilize several important evaluation metrics, including Mean Squared Error (MSE) and Peak Signal-to-Noise Ratio (PSNR). MSE computes the average squared difference between the predicted and real values, indicating the overall accuracy of the model's predictions. PSNR, on the other hand, assesses the quality of the reconstructed images by comparing the maximum possible signal to the noise present in the predictions. An effective model is characterized by a high PNSR value and a low MSE loss value. These metrics offer a comprehensive evaluation of the model's performance, reflecting both accuracy and image quality.

### Hierarchical approach

As mentioned above, for next-state prediction we used two approaches, AE-ConvLSTM and a hierarchical method, which combines four Deep Learning algorithms. These algorithms are the auto-encoders, which are employed to learn efficient representations of complex imaging data, LSTM networks capture long-term dependencies in sequential medical scans, and GANs generate realistic synthetic medical images. This integrated approach leverages the strengths of each algorithm to provide a robust and comprehensive method for disease state prediction.

The proposed model resumes in three stages: 1) Landmarks extraction, 2) Next landmarks prediction, and 3) Pathology next-state image generation.

Landmarks extraction takes an initial image xt as an input, to extract the reference points of the detected pathology. These points will be the input of the next step, to predict the next significant points of the disease.

Finally, the image generation model reconstructs a realistic image from the predicted landmarks and the input frame.

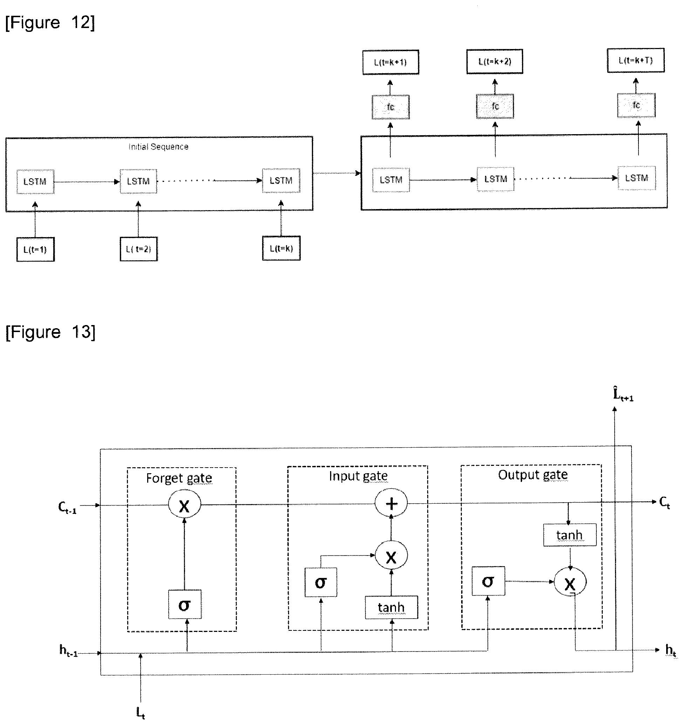

*Landmarks extraction*: Landmarks have been extracted from medical images and organized into a well-defined data structure to facilitate next reference points prediction and image generation. Utilizing landmarks is clearly described in the literature, along with the use of a pre-trained model to extract them, the result is an array containing landmarks, where each row represents a landmark that serves as a reference point in a three-dimensional space (x-axis, y-axis, z-axis), defined by its coordinates. *Next landmarks prediction*: A sequence-to-sequence LSTM network is developed, as illustrated in **Figure 12,** to predict the future positions of spinal landmarks based on a sequence of input positions derived from medical images. **Figure 13** details the used LSTM in the proposed solution.

*Pathology next-state image generation*: A Generative Adversarial Network (GAN) is chosen for pathology next stage image generation. This latter involves two sub-models; a generator and the discriminator. The generator model has the role to generate new information, while the discriminator model detects whether the generated information is real or fake.

## Pathologies specification recognition process using medical images (block 340)

Medical imaging is a powerful diagnostic technique that provides detailed images of the body's internal structures, making it indispensable in identifying various pathologies. In the context of spinal conditions, the ability to accurately recognize and specify the nature of these pathologies from medical images is crucial. This process, known as pathology specification recognition, involves detecting and characterizing abnormalities within the spine to guide effective treatment. Initially, we performed pathology detection using extracted features as mentioned in the beginning of the present detailed description; now, we aim to achieve the same level of detection directly from the images.

The advantages of pathology specification recognition using medical images are substantial, particularly in the prediction and treatment of spinal conditions. We will focus in this section on detecting specific spinal pathologies, such as, extracting the Cobb angle from coronal view images for scoliosis, extracting vertebral body compression ratios for vertebral fractures, and extracting the degree of spinal cord compression for conditions like spinal stenosis. For example in the case of disc herniation pathology, we aim to detect the exact location, type, side, and grade of the herniation from medical images. This detailed recognition is vital because it enables more precise diagnosis and customized treatment plans, leading to better patient outcomes.

## Results verification and validation by the doctor (blocks 400, 410)

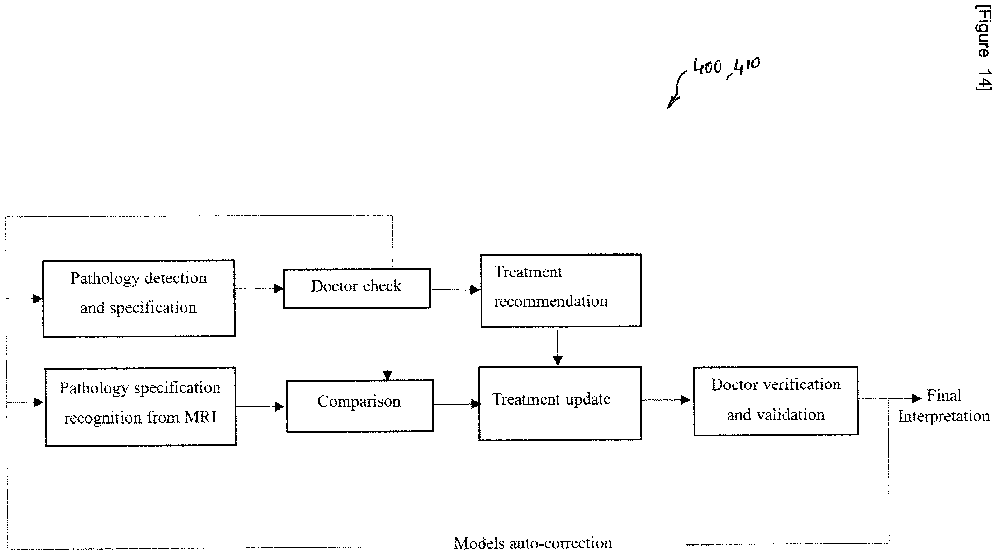

The validation process aims to ensure the accuracy and reliability of pathology detection. This is performed by comparing pathology detection and recognition results, using clinical data (as explained in the beginning of the present detailed description), to the obtained ones using medical image-based analysis (as mentioned just above), as shown in **Figure 14.** This comparison involves multiple steps, such as, the use of techniques to analyze pathology detection and recognition results based on clinical data and medical images, and then results validation.

Both detection and recognition processes produce outputs that include the pathology, its type, localization, and side (if applicable). The comparison step aims to ensure that there is a match between the two processes outputs. If these outputs align, the validation is complete, whereas any discrepancies between these outputs necessitate further review by the doctor.

Technically, the comparison will be performed using language processing models to carry out word-by-word comparisons of the outputs, such as, type vs type, side vs side, etc. The models will analyze each word generated by the two processes, to identify differences.

The doctor reviews the results proposed by the model, and then he can either confirm it or add his interpretations. The system then performs an auto correction and recommends a treatment. The doctor can confirm the suggested treatment or recommend another appropriate one. As more patient cases are incorporated, the model is trained further, thereby generating increasingly effective results.

The method of the invention may be used in many other different application, in particular (but not limitatively):

- patient assessment and monitoring in healthcare facilities, to be used by orthopaedists, neurologists, rheumatologists, general practitioners, radiologists, and physical medicine specialists;
- fraud detection in insurance claims related to occupational diseases, by collecting medical and occupational data of an individual, assessing the correlation between the declared illness and the individual's occupational conditions, and confirming or disputing the cause of the illness, based on predefined criteria and the collected data;
- monitoring and preventing spinal pathologies in heavy vehicle drivers, by measuring vibrations experienced by the driver while operating the vehicle, evaluating the measured vibrations against established health standards for spinal pathologies, and issuing an alert or recommending a mechanism for managing the risk of spinal pathologies, based on the vibration measurements and health standards. The measurement device may be in particular integrated into the driver's seat and capable of transmitting real-time data to the analysis module;
- monitoring the progression of athletes' condition, by tracking the healing process with sensors in order to predict potential anomalies, enabling early intervention to prevent injuries and optimize performance.

## Claims

1. A method for processing physiological data of a spine of a patient, the method comprising the following steps:

   a) collecting and processing clinical data related to the patient's spine;
   b) collecting and processing medical imaging data related to the patient's spine; and

c) reconciling processed clinical data and medical imaging data, for issuing data representative of a status of the patient,

**characterized in that** said processing of clinical data in step a) includes:

a1) pre-processing said clinical data and applying, to pre-processed clinical data, a first spinal pathology detection algorithm;

a2) pre-processing said clinical data and applying, to pre-processed clinical data, a first pathology characterization algorithm, specific to the spinal pathology detected in step a1); and

a3) applying, to data issued by said first algorithms in steps a1) and a2), a therapeutic recommendation algorithm,

**in that** said processing of medical imaging data in step b) includes:

b1) providing, from said medical imaging data, a structural 3D digital model representative of a morphology of the patient's spine;

b2) providing, from said medical imaging data, a predictive digital model representative of a future condition of the patient's spine;

b3) pre-processing said medical imaging data and applying, to pre-processed medical imaging data, a second spinal pathology detection algorithm; and

b4) pre-processing said medical imaging data and applying, to pre-processed medical imaging data, a second pathology characterization algorithm, specific to the spinal pathology detected in step b3);

**and in that** said reconciling in step c) includes:

c1) applying a matching algorithm receiving as inputs the results jointly issued as outputs by said first and second detection algorithms, said first and second pathology characterization algorithms, and said therapeutic recommendation algorithm, respectively applied in steps a1)-a3) and b3)-b4); and

c2) automatically validating or correcting a recommended treatment issued by said therapeutic recommendation algorithm and outputting a validation/invalidation signal to the physician, depending of the result issued by said matching algorithm applied in step c1).

2. The method of claim 1, wherein the specific characterization of a spinal pathology includes determining: a subtype of a pathology, a localization of a pathology with respect to the patient's spine; and a lateralization of a pathology.

[Figure 1]

100 Data collection
CSV
200 Pathology detection
210 Pathology specification recognition
220 Treatment recommendation
Data generation
Data preprocessing
320 Next state prediction of the pathology
300 Image preprocessing
310 Image Segmentation
330 3D reconstruction of the spine
340 Pathologies specification recognition using MRI
400 Results comparison and treatment
410 Results verification and validation

[Figure 2]

[Figure 3]

201     202     203     200   204     205

CSV → | Data preprocessing | → | Data generation | → | Classification | → | Evaluation | → | Deployment |

[Figure 4A]

| | Motor skills (walking on heels) | Muscle strength (walking on toes) | HD detection | Type | Localization HD | HD side | Treatment |
|---|---|---|---|---|---|---|---|
| 1 | | | | | | | |
| 2 | difficult on the right | difficult on the right | HD | Paramedian median foraminal | L5 S1 | Right | Operation |
| 3 | Normal | Normal | HD | Paramedian | L4 L5 | Right | medical treatment |
| 4 | Normal | Normal | HD | Foraminal | L4 L5 | Right | Dissectomy L4L5 |
| 5 | Normal | Normal | HD | Paramedian | L4 L5 | Right | Operation |
| 7 | Normal | Normal | HD | Median | L3 L4 | Left | Operation |
| 9 | Normal | Normal | HD | Median and Paramedian | L4 L5 | Left | medical treatment |

[Figure 4B]

| Motor skills (walking on heels) | Muscle strength (walking on toes) | HD detection | Type | Localization HD | HD side | Treatment |
|---|---|---|---|---|---|---|
| 1 | 1 | 1 | 3 | 4 | 1 | 3 |
| 0 | 0 | 1 | 7 | 2 | 1 | 4 |
| 0 | 0 | 1 | 2 | 2 | 1 | 1 |
| 0 | 0 | 1 | 7 | 2 | 1 | 3 |
| 0 | 0 | 1 | 6 | 1 | 2 | 3 |
| 0 | 0 | 1 | 4 | 4 | 2 | 1 |

[Figure 5]

210

Spinal pathologies detection → Affected → Pathology localization → Pathology type → Pathology side → Treatment recommendation

Spinal pathologies detection → Not affected

[Figure 6]

[Figure 7]

[Figure 8]

[Figure 9]

[Figure 10]

Collected data

300 — Pre-processing

300, 310

Segmented data → Segmentation — 301

ROI identification — 302 → Pathology specification recognition using

Features extraction (landmarks) — 303

Clinical Data → Patient Next State prediction — 304

[Figure 11]

300-1 Image acquisition → 300-2 Image Slicing → 300-3 Image resizing → 300-4 Noise reduction

→ 300-5 Normalization → 300-6 Image labeling → 300-7 Data augmentation

300

[Figure 12]

[Figure 13]

[Figure 14]

400, 410

Pathology detection and specification → Doctor check → Treatment recommendation → Treatment update → Doctor verification and validation → Final Interpretation

Pathology specification recognition from MRI → Comparison → Treatment update

Models auto-correction

**EUROPEAN SEARCH REPORT**

Application Number

EP 24 21 4831

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/157459 A1 (SIEWERDSEN JEFFREY H [US] ET AL) 19 May 2022 (2022-05-19) * abstract; figures 1A, 1B * * paragraph [0014] - paragraph [0015] * * paragraph [0020] * * paragraph [0032] - paragraph [0035] * ----- | 1,2 | INV. G16H20/00 G16H30/40 G16H50/20 G16H50/70 ADD. A61B5/00 |
| A | US 2023/005138 A1 (KUANG XIHE [CN] ET AL) 5 January 2023 (2023-01-05) * the whole document * ----- | 1,2 | G06T7/00 |

TECHNICAL FIELDS
SEARCHED (IPC)

G16H
G06T
A61B

The present search report has been drawn up for all claims

| Place of search | Date of completion of the search | Examiner |
|---|---|---|
| Munich | 5 May 2025 | Kutzarova, Iskrena |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 21 4831

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2025

| Patent document cited in search report | Publication date | Patent family member(s) | Publication date |
|---|---|---|---|
| US 2022157459 A1 | 19-05-2022 | EP 3931838 A1<br>US 2022157459 A1<br>WO 2020180566 A1 | 05-01-2022<br>19-05-2022<br>10-09-2020 |
| US 2023005138 A1 | 05-01-2023 | NONE | |

EPO FORM P0459

For more details about this annex : see Official Journal of the European Patent Office, No. 12/82